# EUROPEAN PATENT SPECIFICATION

(11) **EP 4 301 726 B1**
(45) Date of publication and mention of the grant of the patent: **23.09.2026**
(21) Application number: 22709309.3
(22) Date of filing: 01.03.2022
(51) Int. Cl.: C07C 227/42, C07C 229/52

(54) **SOLIDIFICATION OF HEXYL 2-[4-(DIETHYLAMINO)-2-HYDROXYBENZOYL]BENZOATE**
VERFESTIGUNG VON HEXYL-2-[4-(DIETHYLAMINO)-2-HYDROXYBENZOYL]BENZOAT
SOLIDIFICATION DE HÉXYL 2-[4-(DIÉTHYLAMINO)-2-HYDROXYBENZOYL]BENZOATE

(30) Priority: 03.03.2021 EP 21160493
(43) Date of publication of application: 10.01.2024
(73) Proprietor: BASF SE, 67056 Ludwigshafen am Rhein (DE)
(72) Inventor: SELZER, Daniel Ruben, 67056 Ludwigshafen (DE); KLEINMANN, Eva, 67056 Ludwigshafen (DE); BINDER, Axel, 67056 Ludwigshafen (DE); KRONEMAYER, Helmut, 67056 Ludwigshafen (DE); STAEHLE, Philipp, 67056 Ludwigshafen (DE)
(74) Representative: BASF IP Association
(86) International application number: PCT/EP2022/055088
(87) International publication number: WO 2022/184682

(56) References cited:
- WO-A1-2008/135360

## Description

The present invention relates to a process for the solidification of hexyl 2-[4-(diethylamino)-2-hydroxybenzoyl]benzoate (INCI diethylamino hydroxybenzoyl hexyl benzoate, DHHB), wherein the process comprises a steps (a) of applying a shear rate of from 10 to less than 800 s⁻¹ to liquid hexyl 2-[4-(diethylamino)-2-hydroxybenzoyl]benzoate by stirring the liquid DHHB at a stirring speed of 5 to 200 rpm and (b) adding seed crystals of hexyl 2-[4-(diethylamino)-2-hydroxybenzoyl]benzoate, while applying the shear rate of step (a).

Hexyl 2-[4-(diethylamino)-2-hydroxybenzoyl]benzoate (INCI diethylamino hydroxybenzoyl hexyl benzoate), also referred to as DHHB, is an UV-A filter belonging to the group of benzophenone derivatives. It is sold under the tradename Uvinul A Plus by BASF. It has a melting point of about 54° C.

It is known in the art that solvent-free DHHB melt is hard to crystallize. An undercooled melt can stay in a metastable liquid state for weeks, until it finally crystallizes. An economical solidification process like flaking or pastillation on a cooling belt with such a very slowly crystallizing product is not known, so far. Currently, methods like crystallizing DHHB in tubs or drums and afterwards crushing it are applicable. These methods generally comprise applying high shear rates, which however requires a high energy input. However, these methods have a bad space time yield and lead to non-uniform particle shapes and sizes, which can lead to drawbacks, e.g., regarding its caking properties. WO 2008/135360 A1 describes solidified DHHB in the form of pastilles having small particle sizes as well as processes to prepare such solidified DHHB.

It was therefore an object of the present invention to provide an improved process for the solidification of DHHB. In particular, it was an object of the present invention to provide a process, which has economic advantages in comparison to the prior art methods. In this connection, it was particularly an object of the present invention to provide a process, which needs a reduced energy input and/or provides an improved space time yield. Moreover, it was an object to provide the solidified DHHB in uniform particle shapes and sizes, preferably exhibiting a good flowability and/or good storage stability.

It has surprisingly been found that at least one of the afore-mentioned objects can be achieved by the process of the present invention. In particular, it has surprisingly been found by the inventors of the present invention that applying a low shear rate to a DHHB melt or subcooled melt can provoke in combination with seeding a crystallization of DHHB, and therefore strongly accelerate the crystallization.

The present invention relates in a first aspect to a process for the solidification of hexyl 2-[4-(diethylamino)-2-hydroxybenzoyl]benzoate (INCI diethylamino hydroxybenzoyl hexyl benzoate, DHHB), wherein the process comprises the step of (a) applying a shear rate of from 10 to less than 800 s-1 to liquid hexyl 2-[4-(diethylamino)-2-hydroxybenzoyl]benzoate by stirring the liquid DHHB at a stirring speed of 5 to 200 rpm and (b) adding seed crystals of hexyl 2-[4-(diethylamino)-2-hydroxybenzoyl]benzoate, while applying the shear rate of step (a).

In the following, preferred embodiments of the process of the first aspect are described in further detail. It is to be understood that each preferred embodiment is relevant on its own as well as in combination with other preferred embodiments.

In a preferred embodiment A1 of the first aspect, the liquid hexyl 2-[4-(diethylamino)-2-hydroxybenzoyl]benzoate is provided as a melt or a subcooled melt.

In a preferred embodiment A2 of the first aspect, the melt has a temperature from more than 54 to 70 ° C, more preferably from more than 54 to 65 ° C or the subcooled melt has a temperature from 15 to 54 ° C, more preferably from 20 to 52 ° C.

In a preferred embodiment A3 of the first aspect, the applied shear rate is less than 700 s⁻¹, more preferably less than 500 s⁻¹, and in particular less than 400 s⁻¹ or in the range of from 15 to 700 s⁻¹, preferably from 20 to 600 s⁻¹, and in particular from 25 to 400 s⁻¹.

In a preferred embodiment A4 of the first aspect, the melt or subcooled melt is stirred at a stirring speed of 10 to 150 rpm, and in particular of 15 to 100 rpm.

In a preferred embodiment A5 of the first aspect, the seed crystals are added at a temperature from 30 to 60 ° C, preferably from 40 to less than 54 ° C.

In a preferred embodiment A6 of the first aspect, after addition of the seed crystals the temperature of the hexyl 2-[4-(diethylamino)-2-hydroxybenzoyl]benzoate to be solidified is kept in the range from 15 to less than 54 ° C, preferably from 25 to 52 ° C.

In a preferred embodiment A7 of the first aspect, in step (b) from 0.0001 to 0.1 g, preferably from 0.0005 to 0.05 g, more preferably from 0.001 to 0.03 g, of seed crystals are added per 1 g of the hexyl 2-[4-(diethylamino)-2-hydroxybenzoyl]benzoate to be solidified.

In a preferred embodiment A8 of the first aspect, the seed crystals have a particle size, determined according to sieve analysis, of less than 100000 µm, preferably from 1 to 10000 µm, more preferably from 5 to 5000 µm.

In a preferred embodiment A9 of the first aspect, step (a) is performed in an apparatus, preferably selected from the group consisting of an extruder, a scraped surface heat exchanger, a cooling disc crystallizer, or a stirred vessel, preferably with scraping agitator, which is cooled to a temperature of less than 54 ° C, preferably 40 ° C or less.

In a preferred embodiment A10 of the first aspect, step (a) is performed in a scraped surface heat exchanger and wherein the process further comprises the steps of
(i-1) heating hexyl 2-[4-(diethylamino)-2-hydroxybenzoyl]benzoate until a liquid melt is obtained and
(i-2) feeding the liquid hexyl 2-[4-(diethylamino)-2-hydroxybenzoyl]benzoate obtained in step (i-1) into a scraped surface heat exchanger,
followed by step (a), wherein the liquid hexyl 2-[4-(diethylamino)-2-hydroxybenzoyl]benzoate is stirred by the scraper.

In a preferred embodiment A11 of the first aspect, in step (i-1), a temperature of more than 54 ° C is applied and/or
step (i-2) is performed under heating of the feed to a temperature of more than 54 ° C and/or
the temperature in the scraped surface heat exchanger is less than 54 ° C.

In a preferred embodiment A12 of the first aspect, step (a) is performed in a stirred vessel and the process further comprises the steps of
(ii-1) heating hexyl 2-[4-(diethylamino)-2-hydroxybenzoyl]benzoate until a liquid melt is obtained,
(ii-2) cooling, preferably while stirring, the liquid hexyl 2-[4-(diethylamino)-2-hydroxybenzoyl]benzoate obtained in step (ii-1) to obtain a subcooled melt of hexyl 2-[4-(diethylamino)-2-hydroxybenzoyl]benzoate,
(ii-3) feeding the subcooled melt of hexyl 2-[4-(diethylamino)-2-hydroxybenzoyl]benzoate into a stirred vessel,
followed by step (a).

In a preferred embodiment A13 of the first aspect, in step (ii-1), a temperature of more than 54 ° C is applied and/or
in step (ii-2), the subcooled melt has a temperature in the range from 15 to 54 ° C, preferably from 20 to 52 ° C .

In a second aspect, the present invention relates to solidified hexyl 2-[4-(diethylamino)-2-hydroxybenzoyl]benzoate in the form of
pourable or flowable particles having a particle size of more than 5 to 30 mm,
pastilles having a particle size of more than 5 to 30 mm, or
flakes having a particle size of 1 to 100 mm.

### Detailed Description

Before describing in detail exemplary embodiments of the present invention, definitions important for understanding the present invention are given.

As used in this specification and in the appended claims, the singular forms of "a" and "an" also include the respective plurals unless the context clearly dictates otherwise. In the context of the present invention, the term "approximately" denote an interval of accuracy that a person skilled in the art will understand to still ensure the technical effect of the feature in question. The term typically indicates a deviation from the indicated numerical value of ±15 %, preferably ±10 %, more preferably ±5 %, and in particular ±3 %. It is to be understood that the term "comprising" is not limiting. For the purposes of the present invention the term "consisting of" is considered to be a preferred embodiment of the term "comprising of". If hereinafter a group is defined to comprise at least a certain number of embodiments, this is meant to also encompass a group which preferably consists of these embodiments only. It is to be understood that this invention is not limited to the particular methodology, protocols, reagents etc. described herein as these may vary. It is also to be understood that the terminology used herein is for the purpose of describing particular embodiments only, and is not intended to limit the scope of the present invention that will be limited only by the appended claims. Unless defined otherwise, all technical and scientific terms used herein have the same meanings as commonly understood by one of ordinary skill in the art.

As used herein the term "pourable or flowable particles" refers to any solid form being able to be poured or granulated and which is safe and easy to handle by the processor (e.g. having reduced electrostatic properties when compared to powder).

As used herein the term "pastilles" is a subtype of the pourable or flowable particles, which preferably are hemispherical structures. Pastilles are preferably obtained via the still liquid melt or melt suspension, which can be portioned into small drops and be placed onto the flat surface such that said pastilles are formed. After the melt or melt suspension has crystallized out, the pastilles can be removed and bottled.

As used herein the term "flakes" refers to a specific solid form, which can be obtained by pouring the still liquid melt or melt suspension onto a flat surface, preferably wherein the obtained layer has a thickness of 0.1 to 10 mm, more preferably of 0.2 to 8 mm or of 0.2 to 5 mm or of 0.2 to 2 mm. After the melt or melt suspension has crystallized out, the solid layer is removed from the plane as is customary and bottled, the thin layer usually being comminuted to a desired flake size by breakage.

The production process of pourable or flowable particles, pastilles, and flakes can take place discontinuously (batch process) or continuously, where, in a continuous method, a continuously circulating steel belt, for example, can be used as mold for the purposes of the present invention.

Preferred embodiments regarding the process according to the present invention are described hereinafter. It is to be understood that the preferred embodiments of the invention are preferred alone or in combination with each other.

As indicated above, the present invention relates in one embodiment to a process for the solidification of hexyl 2-[4-(diethylamino)-2-hydroxybenzoyl]benzoate (INCI diethylamino hydroxybenzoyl hexyl benzoate, DHHB), wherein the process comprises the step of
(a) applying a shear rate of from 10 to less than 800 s⁻¹ to liquid hexyl 2-[4-(diethylamino)-2-hydroxybenzoyl]benzoate by stirring the liquid DHHB at a stirring speed of 5 to 200 rpm and
(b) adding seed crystals of hexyl 2-[4-(diethylamino)-2-hydroxybenzoyl]benzoate, while applying the shear rate of step (a).

In a preferred embodiment, the liquid hexyl 2-[4-(diethylamino)-2-hydroxybenzoyl]benzoate is provided as a melt or a subcooled melt.

Hexyl 2-[4-(diethylamino)-2-hydroxybenzoyl]benzoate has a melting point of about 54 ° C. The melting point may vary depending on potential impurities. Therefore, it is to be understood that when referring to the temperature values of the process according to the present invention it is referred to said temperature value ± 2 ° C, preferably ± 1 ° C. For example, if it is referred to the temperature value of the melting point of DHHB of about 54 ° C, it is referred to a temperature range of 54 ° C ± 2 ° C, preferably ± 1 ° C.

In a preferred embodiment, the melt has a temperature from more than about 54 to about 70 ° C, preferably from more than about 54 to about 65 ° C.

In a preferred embodiment, the subcooled melt has a temperature from about 15 to about 54 ° C, preferably from about 20 to about 52 ° C.

In a preferred embodiment, the applied shear rate is less than 700 s⁻¹, more preferably less than 600 s⁻¹, still more preferably less than 500 s⁻¹, and in particular less than 400 s⁻¹. Preferred shear rates are in the range of from 15 to 700 s⁻¹, more preferably from 20 to 600 s⁻¹, and in particular from 25 to 400 s⁻¹. In yet another preferred embodiment, the applied shear rate is in the range of from 20 to less than 800 s⁻¹, preferably from 40 to 700 s⁻¹, more preferably from 50 to 600 s⁻¹, and in particular from 60 to 400 s⁻¹.

As used herein, the term "shear rate" refers to the rate at which progressive shearing deformation is applied to the liquid DHHB. In general, the shear rate may be determined for a fluid flowing between two parallel plates, one moving at a constant speed and the other one stationary based on the following equation: $γ = v / h$ wherein "*γ*" is the shear rate measured in reciprocal seconds, "v" is the velocity of the moving plate, measured in meters per second, and "h" is the distance between the two parallel plates, measured in meters. Based on this principle, the rotational speed and the dimensions of an apparatus used for the application of the shear rate predetermine the shear rate.

In general, high shear rates reduce the time period until nucleation starts, thereby increasing the efficiency of the solidification process. Surprisingly, an efficient solidification process can also be obtained applying low shear rates in combination with seeding. Therefore, efficient solidification of DHHB can be obtained with reduced energy input.

In a preferred embodiment, the melt or subcooled melt is stirred at a stirring speed of less than 200 rpm, more preferably less than 150 rpm, and in particular less than 100 rpm. It is also preferred that the melt or subcooled melt is stirred at a stirring speed of 10 to 150 rpm, preferably of 15 to 100 rpm.

The low shear rate is obtained by stirring the melt or subcooled at a stirring speed of 5 to 200 rpm, preferably of 10 to 150 rpm, more preferably of 15 to 100 rpm.

In a preferred embodiment, the seed crystals are added at a temperature from about 30 to about 60 ° C, more preferably from about 35 to about 55 ° C, and still more preferably from about 40 to less than about 54 ° C.

In a preferred embodiment, after addition of the seed crystals the temperature of the hexyl 2-[4-(diethylamino)-2-hydroxybenzoyl]benzoate to be solidified is kept in the range from about 15 to less than about 54 ° C, preferably from about 25 to about 52 ° C.

In a preferred embodiment, in step (b) from 0.0001 to 0.1 g, preferably from 0.0005 to 0.05 g, more preferably from 0.001 to 0.03 g, of seed crystals are added per 1 g of the hexyl 2-[4-(diethylamino)-2-hydroxybenzoyl]benzoate to be solidified.

In a preferred embodiment, the seed crystals have a particle size, determined according to sieve analysis, of less than 100000 µm, preferably from 1 to 10000 µm, more preferably from 5 to 5000 µm.

In this connection, the determination of the particle size is preferably performed using two sieves, wherein the first sieve has a broader width than the second. Preferably, an amplitude of 1.5 mm is applied and the two sieves are positioned in a Retsch sieve apparatus, wherein the sieve having a mesh having the broader width is located at the upper position. After applying the sample on the upper located sieve, sieving is conducted. The residues are weighed out after intervals within 1 to 20 minutes in order to validate whether the residues of the three obtained fractions change. In general, the distribution of the three fractions does not change any more after 5 to 10 minutes.

Preferably, the determination of the particle size is performed using two sieves, wherein the first sieve has a mesh width of 5 mm and the second sieve has a mesh width of 0.1 mm. Preferably, an amplitude of 1.5 mm is applied and the two sieves are positioned in a Retsch sieve apparatus, wherein the sieve having a mesh width of 5 mm is located at the upper position. After applying the sample on the upper located sieve, sieving is conducted. The residues are weighed out after intervals within 1 to 20 minutes in order to validate whether the residues of the three obtained fractions change. In general, the distribution of the three fractions does not change any more after 5 to 10 minutes. The first fraction comprises particles having a particle size of less than 0.1 mm, the second fraction comprises particles having a particle size of 0.1 to 5 mm, and the third fraction comprises particles having a particle size of more than 5 mm.

In a preferred embodiment, the step (a) is performed in an apparatus, preferably selected from the group consisting of an extruder, a scraped surface heat exchanger, a cooling disc crystallizer, or a stirred vessel, preferably with scraping agitator, which is cooled to a temperature of less than about 54 ° C, preferably about 40 ° C or less.

In this connection it is to be understood that every apparatus, which can be cooled and which enables stirring may be used.

In a preferred embodiment the process is a continuous process.

Preferably, a continuously operated process comprises using a scraped surface heat exchanger and a storage vessel where the DHHB melt can be stored above its melt temperature. A scraped surface heat exchanger, which can be fed with DHHB melt from the storage vessel, is used to generate a melt suspension. In the scraped surface heat exchanger, liquid DHHB is cooled down by means of a cooled internal surface (also referred to as scraped surface) and stirred by the scraper. According to the present invention seed crystals are added. After the onset of crystallization, crystals are generated on a cold internal surface and scraped off by means of a scraper comprised in the scraped surface heat exchanger. During the startup phase, the generated melt suspension is fed back to the storage vessel until the desired solid content in the generated melt suspension is reached. As soon as crystallization starts significantly, an increase of the turbidity of the DHHB melt suspension can be observed (e.g. via the signal of the turbidity probe). Moreover, a color change from brownish to bright yellow can be observed when crystallization starts significantly.

As soon as the desired solid content is reached, the melt suspension can be continuously applied to a maturing belt, preferably a cooling belt (more preferably with multiple temperature zones).

In a preferred embodiment, step (a) is performed in a scraped surface heat exchanger and the process further comprises the steps of
(i-1) heating hexyl 2-[4-(diethylamino)-2-hydroxybenzoyl]benzoate until a liquid melt is obtained and
(i-2) feeding the liquid hexyl 2-[4-(diethylamino)-2-hydroxybenzoyl]benzoate obtained in step (i-1) into a scraped surface heat exchanger,
followed by step (a), wherein the liquid hexyl 2-[4-(diethylamino)-2-hydroxybenzoyl]benzoate is stirred by the scraper.

Preferably, in step (i-1), a temperature of more than about 54 ° C is applied. It is also preferred that in step (i-1), a temperature of from more than about 54 to about 70° C, more preferably from more than about 54 to about 65 ° C is applied.

Preferably, step (i-2) is performed under heating of the feed to a temperature of more than about 54 ° C. It is also preferred that step (i-2) is performed under heating of the feed to a temperature of from more than about 54 to about 70 ° C, more preferably from more than about 54 to about 65° C.

Preferably, the temperature in the scraped surface heat exchanger is less than about 54 ° C, preferably less than about 52° C.

Preferably, the internal surface of the scraped surface heat exchanger has a temperature of less than about 50 ° C, more preferably less than about 40 ° C, still more preferably less than about 30° C, and in particular less than about 20 ° C. It is also preferred that the internal surface of the scraped surface heat exchanger has a temperature of from about 1 to about 50° C, more preferably from about 2 to about 40 ° C, still more preferably from about 3 to about 30° C, and in particular from about 5 to about 20 ° C.

In this connection it is to be understood that the scraped surface heat exchanger is cooled via its internal surface. Therefore, when referred to the temperature in the scraped surface heat exchanger, it is referred to the approximate temperature of the melt/melt suspension, which is cooled via the cooled internal surface. When referred to the temperature of the internal surface it is referred to the temperate of the internal surface of the scraped surface heat exchanger.

In a preferred embodiment, in step (i-1), a temperature of more than about 54 ° C is applied, the step (i-2) is performed under heating of the feed to a temperature of more than about 54 ° C and the temperature in the scraped surface heat exchanger is less than about 54° C.

In a preferred embodiment, step (a) is performed in a stirred vessel and the process further comprises the steps of
(ii-1) heating hexyl 2-[4-(diethylamino)-2-hydroxybenzoyl]benzoate until a liquid melt is obtained,
(ii-2) cooling, preferably while stirring, the liquid hexyl 2-[4-(diethylamino)-2-hydroxybenzoyl]benzoate obtained in step (ii-1) to obtain a subcooled melt of hexyl 2-[4-(diethylamino)-2-hydroxybenzoyl]benzoate,
(ii-3) feeding the subcooled melt of hexyl 2-[4-(diethylamino)-2-hydroxybenzoyl]benzoate into a stirred vessel,
followed by step (a).

Preferably, in step (ii-1), a temperature of more than about 54 ° C is applied. It is also preferred that in step (ii-1), a temperature of from more than about 54 to about 70 ° C, more preferably from more than about 54 to about 65 ° C is applied.

Preferably, in step (ii-2), the subcooled melt has a temperature of less than about 54 ° C. It is also preferred that in step (ii-2), the subcooled melt has a temperature in the range from about 15 to about 54 ° C, preferably from about 20 to about 52 ° C.

In a preferred embodiment, in step (ii-1), a temperature of more than about 54 ° C is applied and in step (ii-2), the subcooled melt has a temperature of less than about 54 ° C.

In a preferred embodiment, step (b) provides a melt suspension.

It is to be understood that according to the present invention the term "melt suspension" denotes a melt comprising solids. For examples a melt suspension of DHHB comprises DHHB in liquid, i.e. molten form, and in solid form.

The melt suspension provided by step (b) may be poured on any suitable container in order to allow the melt suspension to further cool and solidify.

In a preferred embodiment, step (b) provides the hexyl 2-[4-(diethylamino)-2-hydroxybenzoyl]benzoate in the form of solidified strands.

In a preferred embodiment, step (b) provides the hexyl 2-[4-(diethylamino)-2-hydroxybenzoyl]benzoate in the form of a melt suspension, which is solidified by the further steps of
(c) cooling the melt suspension on a maturing belt, preferably a cooling belt, or on a drum flaker at a temperature of less than about 54° C, preferably less than about 40 ° C to obtain a solidified melt and
(d) optionally breaking the solidified melt into flakes or particles.

In a preferred embodiment, step (b) provides the hexyl 2-[4-(diethylamino)-2-hydroxybenzoyl]benzoate in the form of a melt suspension, which is solidified by a further step of
(c-i) forming drops of the melt suspension and cooling them on a maturing belt, preferably a cooling belt, at a temperature of less than about 54 ° C, preferably less than about 40 ° C to obtain solidified pastilles.

In a preferred embodiment, a cooling belt is applied and the cooling belt comprises at least one cooling zone. Preferably, the at least one cooling zone is in the temperature range from about 0 to about 40 ° C, more preferably from about 10 to about 38 ° C, and in particular from about 20 to about 35° C.

Preferably, the cooling belt comprises at least two cooling zones, more preferably wherein the temperature of the first cooling zone is higher than the temperature of the second cooling zone. Preferably, the temperature in the first cooling zone is about 5 ° C, more preferably about 10 ° C, higher than the temperature in the second cooling zone. Preferably, the first cooling zone is in the temperature range from about 15 to about 40 ° C, more preferably from about 22 to about 38 ° C, and the second cooling zone is in the temperature range from about 5 to about 30° C, more preferably from about 10 to about 20 ° C.

In a preferred embodiment, the process is performed as a continuous process, wherein the liquid hexyl 2-[4-(diethylamino)-2-hydroxybenzoyl]benzoate is continuously fed into a scraped surface heat exchanger or an extruder and the hexyl 2-[4-(diethylamino)-2-hydroxybenzoyl]benzoate is continuously collected from the scraped surface heat exchanger or the extruder in the form of solidified strands or in the form of a melt suspension.

If the hexyl 2-[4-(diethylamino)-2-hydroxybenzoyl]benzoate is still not completely solidified after the residence time of the cooling belt a ripening belt can be used subsequent to the cooling belt.

In a second aspect, the present invention relates to solidified hexyl 2-[4-(diethylamino)-2-hydroxybenzoyl]benzoate in the form of
pourable or flowable particles having a particle size of more than 5 to 30 mm,
pastilles having a particle size of more than 5 to 30 mm, or
flakes having a particle size of of 1 to 100 mm.

The pourable or flowable particles have a particle size, determined according to sieve analysis, of more than 5 to 30 mm, and in particular of 10 to 25 mm.

Any suitable method for determining the particle size of the pourable or flowable particles may be applied.

The particle size of the pourable or flowable particles may be determined via sieve analysis. Preferably, the determination of the particle size is performed using two sieves, wherein the first and the second sieves have a mesh width, which are suitable for the determination of e.g. 1 to 30 mm. Preferably, an amplitude of 1.5 mm is applied and the two sieves are positioned in a Retsch sieve apparatus, wherein the sieve having the broader mesh width is located at the upper position. After applying the sample on the upper located sieve, sieving is conducted for 1 to 20 minutes until no change of the distribution of the three fractions is detected.

The particle size of the pourable or flowable particles may also be determined according to caliper for large particles. In this connection it is to be understood that the Feretₘₐₓ. of Feret's diameter is decisive for the particle size.

Sieving is preferably used for of the pourable or flowable particles having a particle size of less than 20 mm, more preferably less than 10, and in particular of 5 mm and less.

Image analysis or caliper are preferably used for the pourable or flowable particles having a particle size of more than 5 mm, more preferably more than 10 mm.

Preferably, the pourable or flowable particles have a bulk density of 0.35 g/mL or more, more preferably of 0.35 to 0.5 g/mL.

In a preferred embodiment, the pastilles have a particle size of 6 to 20 mm.

Any suitable method for determining the particle size of the pastilles may be applied. The particle size of the pastilles may be determined according to image analysis. Therefore, 100 pastilles are randomly selected from the final product. The particle sizes are determined and the average particle diameter is derived therefrom.

The particle size of the pastilles may also be determined according to caliper. In this connection it is to be understood that the Feretₘₐₓ. of Feret's diameter is decisive for the particle size. Using the caliper method is preferred for pastilles having a particle size of more than 5 mm.

Preferably, the pastilles have a bulk density of 0.35 g/mL or more, more preferably of 0.35 to 0.5 g/mL.

The flakes have a particle size of 1 to 100 mm, preferably of 5 to 90 mm, more preferably of more than 5 to 85 mm, still more preferably of 7 to 80 mm, and in particular of 10 to 80 mm.

Any suitable method for determining the particle size of the flakes may be applied.

The particle size of the flakes may be determined according to image analysis. Therefore, 100 flakes are randomly selected from the final product. The particle sizes are determined and the average particle diameter is derived therefrom.

The particle size of the flakes may also be determined according to caliper. In this connection it is to be understood that the Feret_{max.} of Feret's diameter is decisive for the particle size. Using the caliper method is preferred for flakes having a particle size of 5 mm and more.

Preferably, the flakes have a bulk density of 0.35 g/mL or more, more preferably of 0.35 to 0.5 g/mL.

The present invention is further illustrated by the following examples.

### Examples

### Comparative Example 1: Cooling plate experiment with low shear rates, without Seeding

To evaluate the solidification behavior of thin layers (1-3 mm) of a DHHB melt, cooling plate experiments have been performed at a fixed cooling plate temperature of 20 ° C. In the experiment a thin layer of DHHB melt was placed on the surface of the thermostated cooling plate surface (material: stainless steel). To apply a low shear rate of approximately 50 s⁻¹, a spatula was used to stir the liquid DHHB melt gently for several minutes. No crystallization was observed within 2h.

### Comparative Example 2: High Shear Rate without Seeding

The following example is in line with Example 6 of EP 2155660 B1: 5 kg of DHHB is poured in a 5 L aluminum vessel. The melt is stirred by an PTFE propeller stirrer (60 mm diameter), which is stirred by an electrical motor. The melt is stirred at 25 ° C with a stirring speed of 250 rpm (approximately 1000 s⁻¹). After 11h of stirring, melt viscosity increases significantly so that stirring is not possible any more. First crystals are observed after 5h of stirring. Complete solidification is achieved after 24h.

### Comparative Example 3: No Shear Rate with Seeding

The following example is in line with Example 10 of EP 2155660 B1: DHHB seed particles are added to 5kg DHHB melt. The melt temperature at which seed particles (< 100 µm) are added is about 40° C. Afterwards the DHHB melt is allowed to cool down to room temperature. First crystals are observed after 10 days. Complete solidification is achieved after 2 months. The melt is not stirred during the experiment.

### Inventive Example 1: Low Shear Rate with Seeding

A thermostated 1.4 m³ vessel was filled with 1500 kg DHHB melt with a melt temperature of approximately 60 ° C. In a subsequent step, the melt was cooled down in the vessel. During cooling the melt was continuously stirred by means of an anchor type stirrer at 25 rpm. Crystallization progress was monitored by a turbidity probe throughout the experiment. Seeding was applied with 1 wt.-% of solid DHHB granules (particle diameter < 5mm) at a melt temperature of 50 ° C. After 2-4h crystallization started significantly. This could be observed visually by eye and an increase of the turbidity probe signal. Also, a color change of the product from brownish to yellow could be observed. The melt temperature at this point in time was approximately 43 ° C. After the start of crystallization a sample of the melt suspension was poured in a bucket (layer thickness approximately 11 cm) and stored at room temperature. Complete solidification of the sample was observed after approximately 12h. For further process parameters see Table 2.

**Table 2: Process parameters for the crystallization of DHHB in a stirring vessel.**

| **Process Parameter** | **Parameter Range** |
|---|---|
| Volume of stirred vessel | 1.4 m³ |
| Amount of melt in vessel | 1500 kg |
| Shear rate | ~ 100 s⁻¹ |
| Stirring speed | 20 - 30 rpm |
| Stirrer type | anchor type stirrer |
| Crystallization temperature of DHHB melt | ~ 40-50 ° C |
| Added seed mass* | ~ 0.01 g_{Seeds}/g_{Melt} |
| Seeding temperature | < 54° C |
| Time until crystallization onset with seeding | < 2-4h |
| Complete solidification | Approx. 12h |

| | |
|---|---|
| * For seeding, DHHB in the form of pourable or flowable particles were used. | |

### Inventive Example 2: Low Shear Rate with Seeding

A 3L glass crystallizer was filled with approximately 3L of DHHB melt (approximately 3 kg). Afterwards the melt was heated up well above its melting temperature to approximately 60 ° C. In a subsequent step, the melt was cooled down to the desired crystallization temperature by means of a thermostat. The stirring rate of the anchor stirrer was maintained constant at 50 rpm during the experiment. To detect the start of significant crystallization the turbidity and the stirrer torque was recorded continuously throughout the experiment. As soon as the DHHB melt reached 50 ° C, 1 wt.-% DHHB seed particles (30 g) were added to the melt. DHHB in the form of pourable or flowable particles with a particle size of up to 5 mm were used as seed particles. The start of crystallization was observed in less than 3h at a melt temperature of approximately 33 ° C. During the experiment, the melt was continuously cooled down to the desired crystallization temperature of 30 ° C. The start of crystallization could be observed visually by eye, an increase of the turbidity probe signal and an increase of the stirrer torque. Also, a color change of the product from brownish to yellow could be observed. After crystallization started, a sample was taken in a steel tray (layer thickness approximately 7 cm), which completely crystallized at room temperature after approximately 6h.

**Table 2: Process parameters for the crystallization of DHHB in a stirring vessel.**

| **Process Parameter** | **Parameter Range** |
|---|---|
| Volume of stirred vessel | 0,003 m³ |
| Amount of melt in vessel | ~ 3 kg |
| Shear rate | ~ 100 s⁻¹ |
| Stirring speed | 50 rpm |
| Stirrer type | anchor type stirrer |
| Crystallization temperature of DHHB melt | ~ 30-50 ° C |
| Added seed mass* | ~ 0.01 g_{Seeds}/g_{Melt} |
| Seeding temperature | < 54 ° C |
| Time until crystallization onset with seeding | < 3h |
| Complete solidification | Approx. 6h |

| | |
|---|---|
| * For seeding, DHHB in the form of pourable or flowable particles were used. | |

Surprisingly the complete solidification of the DHHB melt suspension can be obtained faster when seed crystals are applied even if lower shear rates are applied.

## Claims

1. A process for the solidification of hexyl 2-[4-(diethylamino)-2-hydroxybenzoyl]benzoate (INCI diethylamino hydroxybenzoyl hexyl benzoate, DHHB), wherein the process comprises the step of
(a) applying a shear rate of from 10 to less than 800 s⁻¹ to liquid hexyl 2-[4-(diethylamino)-2-hydroxybenzoyl]benzoate by stirring the liquid DHHB at a stirring speed of 5 to 200 rpm, and
(b) adding seed crystals of hexyl 2-[4-(diethylamino)-2-hydroxybenzoyl]benzoate, while applying the shear rate of step (a).

2. The process according to claim 1, wherein the liquid hexyl 2-[4-(diethylamino)-2-hydroxybenzoyl]benzoate is provided as a melt or a subcooled melt.

3. The process according to claim 2, wherein the melt has a temperature from more than 54 to 70 °C, preferably from more than 54 to 65 °C or
the subcooled melt has a temperature from 15 to 54 °C, preferably from 20 to 52 °C.

4. The process according to any one of claims 1 to 3, wherein the applied shear rate is less than 700 s⁻¹, preferably less than 500 s⁻¹, and in particular less than 400 s⁻¹ or
in the range of from 15 to 700 s⁻¹, preferably from 20 to 600 s⁻¹, and in particular from 25 to 400 s⁻¹.

5. The process according to any one of claims 1 to 4, wherein the melt or subcooled melt is stirred at a stirring speed of 10 to 150 rpm, preferably of 15 to 100 rpm.

6. The process according to any one of claims 1 to 5, wherein the seed crystals are added at a temperature from 30 to 60 °C, preferably from 40 to less than 54 °C.

7. The process according to any one of claims 1 to 6, wherein after addition of the seed crystals the temperature of the hexyl 2-[4-(diethylamino)-2-hydroxybenzoyl]benzoate to be solidified is kept in the range from 15 to less than 54 °C, preferably from 25 to 52 °C.

8. The process according to any one of claims 1 to 7, wherein in step (b) from 0.0001 to 0.1 g, preferably from 0.0005 to 0.05 g, more preferably from 0.001 to 0.03 g, of seed crystals are added per 1 g of the hexyl 2-[4-(diethylamino)-2-hydroxybenzoyl]benzoate to be solidified.

9. The process according to any one of claims 1 to 8, wherein the seed crystals have a particle size, determined according to sieve analysis, of less than 10000, preferably from 1 to 100000 µm, more preferably from 5 to 5000 µm.

10. The process according to any one of claims 1 to 9, wherein the step (a) is performed in an apparatus, preferably selected from the group consisting of an extruder, a scraped surface heat exchanger, a cooling disc crystallizer, or a stirred vessel, preferably with scraping agitator, which is cooled to a temperature of less than 54 °C, preferably 40 °C or less.

11. The process according to any one of claims 1 to 10, wherein the step (a) is performed in a scraped surface heat exchanger and wherein the process further comprises the steps of
(i-1) heating hexyl 2-[4-(diethylamino)-2-hydroxybenzoyl]benzoate until a liquid melt is obtained and
(i-2) feeding the liquid hexyl 2-[4-(diethylamino)-2-hydroxybenzoyl]benzoate obtained in step (i-1) into a scraped surface heat exchanger,
followed by step (a), wherein the liquid hexyl 2-[4-(diethylamino)-2-hydroxybenzoyl]benzoate is stirred by the scraper.

12. The process according to claim 11, wherein, in step (i-1), a temperature of more than 54 °C is applied and/or
wherein the step (i-2) is performed under heating of the feed to a temperature of more than 54 °C and/or
wherein the temperature in the scraped surface heat exchanger is less than 54 °C.

13. The process according to any one of claims 1 to 10, wherein the step (a) is performed in a stirred vessel and wherein the process further comprises the steps of
(ii-1) heating hexyl 2-[4-(diethylamino)-2-hydroxybenzoyl]benzoate until a liquid melt is obtained,
(ii-2) cooling, preferably while stirring, the liquid hexyl 2-[4-(diethylamino)-2-hydroxybenzoyl]benzoate obtained in step (ii-1) to obtain a subcooled melt of hexyl 2-[4-(diethylamino)-2-hydroxybenzoyl]benzoate,
(ii-3) feeding the subcooled melt of hexyl 2-[4-(diethylamino)-2-hydroxybenzoyl]benzoate into a stirred vessel,
followed by step (a).

14. The process according to claim 13, wherein, in step (ii-1), a temperature of more than 54 °C is applied and/or
wherein, in step (ii-2), the subcooled melt has a temperature in the range from 15 to 54 °C, preferably from 20 to 52 °C .

15. Solidified hexyl 2-[4-(diethylamino)-2-hydroxybenzoyl]benzoate in the form of pourable or flowable particles having a particle size of more than 5 to 30 mm,
pastilles having a particle size of more than 5 to 30 mm, or
flakes having a particle size of 1 to 100 mm.

## Patentansprüche

1. Verfahren zur Verfestigung von 2-[4-(Diethylamino)-2-hydroxybenzoyl]benzoesäurehexylester (INCI Diethylamino hydroxybenzoyl hexyl benzoate, DHHB), wobei das Verfahren den
Schritt umfasst
(a) Anlegen einer Scherrate von 10 bis weniger als 800 s⁻¹ an flüssigen 2-[4-(Diethylamino)-2-hydroxybenzoyl]benzoesäurehexylester durch Rühren des flüssigen DHHB bei einer Rührgeschwindigkeit von 5 bis 200 rpm, und
(b) Zugabe von Impfkristallen von 2-[4-(Diethylamino)-2-hydroxybenzoyl]benzoesäurehexylester unter Anlegen der Scherrate aus Schritt (a).

2. Verfahren gemäß Anspruch 1, wobei der flüssige 2-[4-(Diethylamino)-2-hydroxybenzoyl]benzoesäurehexylester als Schmelze oder unterkühlte Schmelze bereitgestellt wird.

3. Verfahren gemäß Anspruch 2, wobei die Schmelze eine Temperatur von mehr als 54 bis 70 °C, vorzugsweise von mehr als 54 bis 65 °C aufweist, oder
die unterkühlte Schmelze eine Temperatur von 15 bis 54 °C, vorzugsweise von 20 bis 52 °C aufweist.

4. Verfahren gemäß einem der Ansprüche 1 bis 3, wobei die angelegte Scherrate weniger als 700 s⁻¹, vorzugsweise weniger als 500 s⁻¹, und insbesondere weniger als 400 s⁻¹ beträgt, oder
im Bereich von 15 bis 700 s⁻¹, vorzugsweise von 20 bis 600 s⁻¹, und insbesondere von 25 bis 400 s⁻¹ liegt.

5. Verfahren gemäß einem der Ansprüche 1 bis 4, wobei die Schmelze oder unterkühlte Schmelze bei einer Rührgeschwindigkeit von 10 bis 150 rpm, vorzugsweise von 15 bis 100 rpm gerührt wird.

6. Verfahren gemäß einem der Ansprüche 1 bis 5, wobei die Impfkristalle bei einer Temperatur von 30 bis 60 °C, vorzugsweise von 40 bis weniger als 54 °C zugegeben werden.

7. Verfahren gemäß einem der Ansprüche 1 bis 6, wobei nach Zugabe der Impfkristalle die Temperatur des zu verfestigenden 2-[4-(Diethylamino)-2-hydroxybenzoyl]benzoesäurehexylesters im Bereich von 15 bis weniger als 54 °C, vorzugsweise von 25 bis 52°C gehalten wird.

8. Verfahren gemäß einem der Ansprüche 1 bis 7, wobei in Schritt (b) von 0,0001 bis 0,1 g, vorzugsweise von 0,0005 bis 0,05 g, besonders bevorzugt von 0,001 bis 0,03 g Impfkristalle pro 1 g des zu verfestigenden 2-[4-(Diethylamino)-2-hydroxybenzoyl]benzoesäurehexylesters zugegeben werden.

9. Verfahren gemäß einem der Ansprüche 1 bis 8, wobei die Impfkristalle eine Partikelgröße, bestimmt gemäß Siebanalyse, von weniger als 10000, vorzugsweise von 1 bis 100000 µm, besonders bevorzugt von 5 bis 5000 µm aufweisen.

10. Verfahren gemäß einem der Ansprüche 1 bis 9, wobei Schritt (a) in einer Vorrichtung durchgeführt wird, die vorzugsweise ausgewählt ist aus der Gruppe bestehend aus einem Extruder, einem Kratzkühler, einem Kühlscheibenkristallisator oder einem Rührbehälter, vorzugsweise mit Kratzrührer, der auf eine Temperatur von weniger als 54 °C, vorzugsweise 40 °C oder weniger gekühlt wird.

11. Verfahren gemäß einem der Ansprüche 1 bis 10, wobei Schritt (a) in einem Kratzkühler durchgeführt wird und wobei das Verfahren ferner die Schritte umfasst
(i-1) Erhitzen von 2-[4-(Diethylamino)-2-hydroxybenzoyl]benzoesäurehexylester, bis eine flüssige Schmelze erhalten wird, und
(i-2) Einleiten des in Schritt (i-1) erhaltenen flüssigen 2-[4-(Diethylamino)-2-hydroxybenzoyl]benzoesäurehexylesters in einen Kratzkühler,
gefolgt von Schritt (a), wobei der flüssige 2-[4-(Diethylamino)-2-hydroxybenzoyl]benzoesäurehexylester durch den Kratzer gerührt wird.

12. Verfahren gemäß Anspruch 11, wobei in Schritt (i-1) eine Temperatur von mehr als 54°C angelegt wird und/oder
wobei Schritt (i-2) unter Erhitzen des Zulaufs auf eine Temperatur von mehr als 54°C durchgeführt wird und/oder
wobei die Temperatur im Kratzkühler weniger als 54 °C beträgt.

13. Verfahren gemäß einem der Ansprüche 1 bis 10, wobei Schritt (a) in
einem Rührbehälter durchgeführt wird und wobei das Verfahren ferner die Schritte umfasst
(ii-1) Erhitzen von 2-[4-(Diethylamino)-2-hydroxybenzoyl]benzoesäurehexylester, bis eine flüssige Schmelze erhalten wird,
(ii-2) Abkühlen, vorzugsweise unter Rühren, des in Schritt (ii-1) erhaltenen flüssigen 2-[4-(Diethylamino)-2-hydroxybenzoyl]benzoesäurehexylesters, um eine unterkühlte Schmelze von 2-[4-(Diethylamino)-2-hydroxybenzoyl]benzoesäurehexylester zu erhalten,
(ii-3) Einleiten der unterkühlten Schmelze von 2-[4-(Diethylamino)-2-hydroxybenzoyl]benzoesäurehexylester in einen Rührbehälter,
gefolgt von Schritt (a).

14. Verfahren gemäß Anspruch 13, wobei in Schritt (ii-1) eine Temperatur von mehr als 54°C angelegt wird und/oder
wobei in Schritt (ii-2) die unterkühlte Schmelze eine Temperatur im Bereich von 15 bis 54 °C, vorzugsweise von 20 bis 52 °C aufweist.

15. Verfestigter 2-[4-(Diethylamino)-2-hydroxybenzoyl]benzoesäurehexylester in Form von schüttfähigen oder fließfähigen Partikeln mit einer Partikelgröße von mehr als 5 bis 30 mm, Pastillen mit einer Partikelgröße von mehr als 5 bis 30 mm, oder
Flocken mit einer Partikelgröße von 1 bis 100 mm.

## Revendications

1. Un procédé pour la solidification du 2-[4-(diéthylamino)-2-hydroxybenzoyl]benzoate d'hexyle (INCI diéthylamino hydroxybenzoyl hexyl benzoate, DHHB), dans lequel le procédé comprend l'étape de
(a) l'application d'un taux de cisaillement de 10 à moins de 800 s⁻¹ au 2-[4-(diéthylamino)-2-hydroxybenzoyl]benzoate d'hexyle liquide en agitant le DHHB liquide à une vitesse d'agitation de 5 à 200 tr/min, et
(b) l'ajout de cristaux germes de 2-[4-(diéthylamino)-2-hydroxybenzoyl]benzoate d'hexyle, tout en appliquant le taux de cisaillement de l'étape (a).

2. Le procédé selon la revendication 1, dans lequel le 2-[4-(diéthylamino)-2-hydroxybenzoyl]benzoate d'hexyle liquide est fourni sous forme de masse fondue ou de masse fondue sous-refroidie.

3. Le procédé selon la revendication 2, dans lequel la masse fondue a une température allant de plus de 54 à 70 °C, de préférence de plus de 54 à 65 °C, ou
la masse fondue sous-refroidie a une température de 15 à 54 °C, de préférence de 20 à 52 °C.

4. Le procédé selon l'une quelconque des revendications 1 à 3, dans lequel le taux de cisaillement appliqué est inférieur à 700 s⁻¹, de préférence inférieur à 500 s⁻¹, et en particulier inférieur à 400 s⁻¹ ou
dans la plage de 15 à 700 s⁻¹, de préférence de 20 à 600 s⁻¹, et en particulier de 25 à 400 s⁻¹.

5. Le procédé selon l'une quelconque des revendications 1 à 4, dans lequel la masse fondue ou la masse fondue sous-refroidie est agitée à une vitesse d'agitation de 10 à 150 tr/min, de préférence de 15 à 100 tr/min.

6. Le procédé selon l'une quelconque des revendications 1 à 5, dans lequel les cristaux germes sont ajoutés à une température de 30 à 60 °C, de préférence de 40 à moins de 54 °C.

7. Le procédé selon l'une quelconque des revendications 1 à 6, dans lequel après l'addition des cristaux germes la température du 2-[4-(diéthylamino)-2-hydroxybenzoyl]benzoate d'hexyle à solidifier est maintenue dans la plage de 15 à moins de 54 °C, de préférence de 25 à 52 °C.

8. Le procédé selon l'une quelconque des revendications 1 à 7, dans lequel à l'étape (b), de 0,0001 à 0,1 g, de préférence de 0,0005 à 0,05 g, plus préférablement de 0,001 à 0,03 g, de cristaux germes sont ajoutés par 1 g de 2-[4-(diéthylamino)-2-hydroxybenzoyl]benzoate d'hexyle à solidifier.

9. Le procédé selon l'une quelconque des revendications 1 à 8, dans lequel les cristaux germes ont une taille de particule, déterminée selon l'analyse par tamisage, inférieure à 10000, de préférence de 1 à 100000 µm, plus préférablement de 5 à 5000 µm.

10. Le procédé selon l'une quelconque des revendications 1 à 9, dans lequel l'étape (a) est réalisée dans un appareil, de préférence choisi dans le groupe constitué d'une extrudeuse, d'un échangeur de chaleur à surface raclée, d'un cristalliseur à disque de refroidissement, ou d'une cuve agitée, de préférence avec un agitateur racleur, qui est refroidi à une température inférieure à 54 °C, de préférence 40 °C ou moins.

11. Le procédé selon l'une quelconque des revendications 1 à 10, dans lequel l'étape (a) est réalisée dans un échangeur de chaleur à surface raclée et dans lequel le procédé comprend en outre les étapes de
(i-1) chauffage du 2-[4-(diéthylamino)-2-hydroxybenzoyl]benzoate d'hexyle jusqu'à l'obtention d'une masse fondue liquide et
(i-2) alimentation de l'échangeur de chaleur à surface raclée avec le 2-[4-(diéthylamino)-2-hydroxybenzoyl]benzoate d'hexyle liquide obtenu à l'étape (i-1),
suivies de l'étape (a), dans laquelle le 2-[4-(diéthylamino)-2-hydroxybenzoyl]benzoate d'hexyle liquide est agité par le racleur.

12. Le procédé selon la revendication 11, dans lequel, à l'étape (i-1), une température supérieure à 54°C est appliquée et/ou
dans lequel l'étape (i-2) est réalisée avec chauffage de l'alimentation à une température supérieure à 54°C et/ou
dans lequel la température dans l'échangeur de chaleur à surface raclée est inférieure à 54 °C.

13. Le procédé selon l'une quelconque des revendications 1 à 10, dans lequel l'étape (a) est réalisée dans une cuve agitée et dans lequel le procédé comprend en outre les étapes de
(ii-1) chauffage du 2-[4-(diéthylamino)-2-hydroxybenzoyl]benzoate d'hexyle jusqu'à l'obtention d'une masse fondue liquide,
(ii-2) refroidissement, de préférence sous agitation, du 2-[4-(diéthylamino)-2-hydroxybenzoyl]benzoate d'hexyle liquide obtenu à l'étape (ii-1) pour obtenir une masse fondue sous-refroidie de 2-[4-(diéthylamino)-2-hydroxybenzoyl]benzoate d'hexyle,
(ii-3) alimentation de la cuve agitée avec la masse fondue sous-refroidie de 2-[4-(diéthylamino)-2-hydroxybenzoyl]benzoate d'hexyle,
suivies de l'étape (a).

14. Le procédé selon la revendication 13, dans lequel, à l'étape (ii-1), une température supérieure à 54 °C est appliquée et/ou
dans lequel, à l'étape (ii-2), la masse fondue sous-refroidie a une température dans la plage de 15 à 54 °C, de préférence de 20 à 52 °C.

15. 2-[4-(diéthylamino)-2-hydroxybenzoyl]benzoate d'hexyle solidifié sous forme de particules versables ou coulantes ayant une taille de particule de plus de 5 à 30 mm, de pastilles ayant une taille de particule de plus de 5 à 30 mm, ou de flocons ayant une taille de particule de 1 à 100 mm.
